(19) **Europäisches Patentamt**
**European Patent Office**
**Office européen des brevets**

(11) **EP 1 961 824 A1**

(12) **EUROPEAN PATENT APPLICATION**
published in accordance with Art. 158(3) EPC

(43) Date of publication:
**27.08.2008 Bulletin 2008/35**

(51) Int Cl.:
*C12Q 1/02* (2006.01)     *G01N 21/27* (2006.01)
*G01N 21/64* (2006.01)     *G01N 33/15* (2006.01)
*G01N 33/50* (2006.01)     *G01N 33/543* (2006.01)

(21) Application number: **06834714.5**

(22) Date of filing: **14.12.2006**

(86) International application number:
**PCT/JP2006/324960**

(87) International publication number:
**WO 2007/069692 (21.06.2007 Gazette 2007/25)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.12.2005 JP 2005360458**

(71) Applicant: **Kyushu University, National University Corporation Higashi-ku Fukuoka-shi Fukuoka 812-8581 (JP)**

(72) Inventors:
• **ONA, Toshihiro,**
**c/o Kyushu University**
**Fukuoka-shi**
**Fukuoka 812-8581 (JP)**
• **KOSAIHIRA, Atsushi,**
**c/o Kyushu University**
**Fukuoka-shi**
**Fukuoka 812-8581 (JP)**

(74) Representative: **HOFFMANN EITLE Patent- und Rechtsanwälte Arabellastrasse 4 81925 München (DE)**

(54) **MONITORING OF INTRACELLULAR MITOCHONDRIAL LOCALIZATION**

(57)     An object is a method of detecting changes in mitochondrial polarized state in a living cell.

Changes in surface plasmon resonance angle resulting from changes in mitochondrial polarized state of the living cells are detected by use of a surface plasmon resonance device. Alternatively, one or plural agents are administered to the living cells, and changes in surface plasmon resonance angle resulting from changes in mitochondrial polarized state are detected. The step of detecting changes in surface plasmon resonance angle resulting from changes in mitochondrial polarized state can be realized by the step of detecting changes in surface plasmon resonance angle during a period in which the changes only result from changes in mitochondrial polarized state, and it is preferable that the changes be detected during a period that comes after a lapse of 20 minutes or longer after the agent(s) was administered, preferably after a lapse of 30 minutes or longer, more preferably after a lapse of 35 minutes or longer.

Figure 7

EP 1 961 824 A1

**Description**

[TECHNICAL FIELD]

The present invention relates to a method of measuring a mitochondrial polarized state in real time, without labeling, by use of a surface plasmon resonance (SPR) sensor. The method is useful for monitoring biological phenomena related to a mitochondrial polarized state and in development of drugs related to a mitochondrial polarized state. For example, the method can be employed for screening anticancer agents, fat burning agents, or drugs for diabetes, in monitoring cell activity including senescence, and in quantification of effects of drugs.

[BACKGROUND OF THE INVENTION]

A mitochondrion is a cell organelle that exists in most organisms, including plants and animals, that have eukaryotic cells. Mitochondria play an important role in vital energy metabolism of living organisms. Thus, behavior of mitochondria is closely related to a cell state at a time of cell division, at a time of cell death, during senescence, and in various diseases including cancer, diabetes, and obesity. One of the indicators of the cell state is a change in mitochondrial polarized state. For instance, it is known that a level of mitochondrial polarization decreases in senescent cells, whereas it is high in cells with high division activity (Biological Signals and Receptors 2001; 10: 176-188). Further, a mitochondrial polarized state changes in a variety of ways in reaction to different cellular responses to, for instance, administration of an anticancer agent to cancer cells, exposure of cells to a toxic agent, or fat-burning of fat cells. (Anticancer: Apoptosis 2005; 10: 687-705. Toxic agent: Hepatology 2000; 31: 1141-1152, and Toxicological Sciences 2005; 86(2): 436-443. Lipid metabolism: Biophysical journal 2002; 82(1): 1673 part 2, and FEBS Letters 1984; 170(1): 181-185.) Thus, monitoring mitochondrial polarized states is used as one of the effective indicators in diagnosis of various diseases and in development of drugs.

To monitor an intercellular mitochondrial polarized state, the following method is generally employed. Fluorescent dye that is responsive to electric potential is introduced into cells. The dye forms aggregates in mitochondria having a high electric potential and causes a change in fluorescence wavelength and, in response to a change in electric potential, causes a change in fluorescence intensity. Such changes can be detected using a fluorescent microscope, flow cytometry, a spectrophotometer, or the like.

Meanwhile, a surface plasmon resonance (SPR) device enables determination of changes in a resonance angle by use of surface plasmon resonance phenomena. Changes in resonance angle are dependent on changes in dielectric constant in a vicinity of a surface of a gold layer of a sensor section. In the SPR device, a target is immobilized to the gold layer, and a ligand for the target is provided. Each individual biomolecule has an intrinsic dielectric constant. When the target immobilized to the gold layer binds to the ligand, a complex is formed to cause a change in dielectric constant. Thus, information on a presence or absence of bindings between biomolecules, a quantity of binding, a speed of binding, and the like can be obtained by tracking the dielectric constant of the surface of the metal layer.

With the SPR device, not only biomolecules such as proteins, but also living cells can be determined.

For instance, JP 2002-85089 A discloses a method of evaluating effects of external stimuli on physiological activity of living cells by use of a surface plasmon resonance device. A feature of the method is that the evaluation of effect of external stimuli on cell activity utilizes, as indicators, not only signals (first signal) during a period in which the cells are stimulated, but also second signals, which appear following the first signals. Concretely, when ligands bind to living cells immobilized in the SPR device, a baseline increases in proportion to the quantity thereof and is stabilized (first signal). If the ligands exhibit a physiological activity on the cells, an increase or periodical change in baseline (second signal) that distinctly differs from a mere signal of binding is observed following the initial signals. Since the second signals appear only when ligands that are confirmed to have physiological activity are added, the second signals are considered to reflect some sort of biological response triggered when ligands bind to living cells. Therefore, according to the publication referred to above, the method enables accurate evaluation of effects of external stimuli on physiological activity of cells. The publication describes that a preferred aspect of the method is to measure, as the second signals, signals that appear after the external stimuli are removed. The publication also includes Examples determining (1) reaction of CTLL-2 cells (floating cell) and IL-2, (2) reaction of Papilla cells (mesenchymal cell) and bFGF, and (3) reaction of mast cells (mastocyte) to an antigen. With regard to (1), the first signals were obtained during a period of several minutes when stimulation with IL-2 was carried out, and second signals appearing thereafter were obtained; although the second signals were not observed when a reagent that did not bind to CTLL-2 cells was added. The publication concludes that the SPR devices captured some sort of phenomena that occurs when cells bind to IL-2. With regard to (2), when bFGF and a phosphorylation inhibitor (SU4984) were injected, the signals reached a plateau at 10 minutes after injection. On the other hand, when only bFGF was injected, the signals kept increasing. The publication concludes that the foregoing results reflect that signal transduction that would be triggered when bFGF binds was suppressed by the phosphorylation inhibitor. With regard to (3), strong signals (second signal) reflecting activation associated with a specific antigen-antibody reaction were observed in IgE-sensitized mast cells, but no characteristic change was observed in naive mast cells. When DNP-lysine, which would bind to IgE but would not cause cell activation, was added, the first signals associated with binding of DNP-lysine were observed, but the second signals were not observed. The publication concludes therefrom that the method enables prompt measurement of cell activation.

JP 2002-85089 A discloses that, in a surface plasmon resonance device, the second signals reflect a particular phenomenon that is proved, by determination in an existing reaction system, to definitely occur.

JP 2005-17081 A discloses a method of screening an agent with antitumor activity by use of an SPR device. In the method, a target reagent is caused to act on cancer cells, and surface plasmon resonance is measured. A rate of change in surface plasmon resonance during a time period in which the rate of change with respect to time is stable is obtained, and the level of antitumor activity of the target reagent is evaluated on the basis of the obtained rate of change in surface plasmon resonance. In the Example, a gradient of a curve obtained on the basis of the surface plasmon resonance response to quercetin with antiproliferation action was obtained for the five-minute period between 2700 and 3000 seconds after the measurement was started (the reagent was administered 600 seconds after the measurement was started). Further, a survival rate with respect to the reagent was obtained 48 hours after the administration of the reagent by trypan blue staining. Since there was a correlation between the gradient and the survival rate, the publication concludes that cell viability, that is to say, antitumor activity can be evaluated quantitatively by measuring the range of change in surface plasmon resonance response during a predetermined short period.

[DISCLOSURE OF INVENTION]

[TECHNICAL PROBLEM]

However, the foregoing methods of detecting depolarization of mitochondria by use of a fluorescent microscope, a flow cytometry, a spectrophotometer, or the like have the following problems. Since the methods need introduction of fluorescent reagents, demanding operations become necessary, and the introduction of the fluorescent reagents may affect cells. Another problem is low reproducibility due to inequality in the amount of fluorescent reagent introduced. The methods also involve a problem in that detection takes time.

There is also another method in which a mitochondrion alone is separated from a cell and dispersed in a solution, and an electric potential is measured directly without labeling by use of electrodes or the like. This method, however, is not suitable for monitoring a response of a mitochondrion in a living body, because a response of a mitochondrion within a cell to an external stimulus is entirely different from that of a mitochondrion outside of a cell.

[TECHNICAL SOLUTION]

The inventors of the present invention have diligently studied methods of monitoring responses of living cells to a variety of external stimuli. As a result, they found that changes in mitochondrial polarized state can be detected by use of a surface plasmon resonance angle device. With this finding, the inventors have completed the present invention.

The present invention provides the following methods:

(1) a method of detecting a change in mitochondrial polarized state in a living cell, which method includes detecting, by use of a surface plasmon resonance device, a change in surface plasmon resonance angle resulting from a change in mitochondrial polarized state of the living cell; and

(2) a method of detecting a change in mitochondrial polarized state in a living cell, including:

administering an agent to the living cell; and
detecting, after the agent is administered to the living cell, a change in surface plasmon resonance angle resulting from a change in mitochondrial polarized state.

[ADVANTAGEOUS EFFECT OF THE INVENTION]

The present invention reveals that a change in mitochondrial polarized state is detectable in the form of a change in surface plasmon resonance angle. It is also revealed that a speed of change in polarization of the mitochondrion is controllable by controlling a speed of pH change during measurement.

The present invention solves the problems of the conventional techniques, such as complication associated with introduction of a fluorescent reagent, impacts of introduction of a fluorescent reagent on cells, and low reproducibility of fluorescence intensity due to inequality in the amount of introduced reagent. Further, the present invention enables changes in mitochondrial polarized state to be detected in a shorter period of time than that in the conventional techniques.

[BRIEF DISCRIPTION OF THE DRAWINGS]

[Figure 1] Figure 1 is a diagram showing a device employed in the Examples.

[Figure 2] Figure 2 is a graph showing the results of measurement of changes in mitochondrial polarized state caused by fenofibrate, which is an agent for activating lipid metabolism, using a SPR sensor.

[Figure 3] Figure 3 is a graph showing the results of measurement of changes in mitochondrial polarized state caused by fenofibrate, which is an agent for activating lipid metabolism, using a fluorescent microscope for observation.

[Figure 4] Figure 4 is a graph showing a correlation between a time rate of change in SPR signals (35-45 minutes after administration of a reagent) and a time rate of change in fluorescence intensity (35-45 minutes after the administration of the reagent) in Example 2.

[Figure 5] Figure 5 is a graph showing the results of measurement of changes in mitochondrial depolarization at the time of administration of an apoptosis-inducing agent, using an SPR sensor.

[Figure 6] Figure 6 is a graph showing the results of measurement of changes in mitochondrial depolarization at the time of administration of an apoptosis-inducing agent, using a fluorescent microscope for observation.

[Figure 7] Figure 7 is a graph showing a correlation between the time rate of change (35-45 minutes after the administration of the reagent) and the time rate of change in fluorescence intensity (35-45 minutes after the administration of the reagent).

[Figure 8] Figure 8 is a graph showing changes in mitochondrial depolarization as a result of administration of an apoptosis-inducing agent (quercetin), in a case of suppressing mitochondrial depolarization (observation with a fluorescent microscope).

[Figure 9] Figure 9 is a graph showing changes in mitochondrial depolarization as a result of administration of an apoptosis-inducing agent (*trans*-resveratrol), in the case of suppressing mitochondrial depolarization (observation with a fluorescent microscope).

[Figure 10] Figure 10 is a graph showing changes in mitochondrial depolarization as a result of administration of an apoptosis-inducing agent (quercetin), in the case of suppressing mitochondrial depolarization (SPR sensor).

[Figure 11] Figure 11 shows the results of changes in mitochondrial depolarization as a result of administration of an apoptosis-inducing agent (*trans*-resveratrol), in the case of suppressing mitochondrial depolarization (SPR sensor).

[Figure 12] Figure 12 is a graph showing cell viability 48 hours after the administration of apoptosis-inducing agents (quercetin, *trans*-resveratrol), either alone or in combination (counting the number of cells).

[Figure 13] Figure 13 is a graph showing changes in SPR angle over time at the time of administration of apoptosis-inducing agents (quercetin, *trans*-resveratrol), either alone or in combination (SPR sensor).

[Figure 14] Figure 14 is a graph showing a correlation between the speed of change in SPR angle and the cell viability at the time of administration of apoptosis-inducing agents (quercetin, *trans*-resveratrol), either alone or in combination.

[Figure 15] Figure 15 is a graph showing cell viability 48 hours after the administration of siRNA (counting the number of cells).

[Figure 16] Figure 16 is a graph showing changes in SPR angle over time at the time of the administration of siRNA (SPR sensor).

[Figure 17] Figure 17 is a graph showing a correlation between the speed of change in SPR angle and the cell viability at the time of the administration of siRNA.

[Figure 18] Figure 18 is a graph showing changes in SPR angle over time under respective conditions of $CO_2$ concentrations (SPR sensor).

[Figure 19] Figure 19 is a graph showing a correlation between a $CO_2$ concentration and a speed of change in mitochondrial membrane potential during a period between 35 and 40 minutes (observation with a fluorescent microscope).

[Figure 20] Figure 20 is a graph showing a correlation between a speed of pH change and a speed of change in mitochondrial membrane potential during a period between 35 and 40 minutes.

[EMBODIMENT]

A "living cell" in a method of the present invention is not particularly limited, as long as it is a living cell having a mitochondrion. Examples of the living cell include normal cells, cancer cells, fertilized ova, clonal cells of plants and animals, ES cells, and cancer stem cells, and they may be cultured cells or cells derived from tissues. The living cell for use can be selected according to the purpose of detecting changes in mitochondrial polarized state in the living cell. For instance, cancer cells or cancer stem cells can be employed to evaluate anticancer agents. Hepatic cells can be employed for lipid metabolism. Fertilized ova or ES cells can be employed to monitor differentiation. Plant cells can be employed to evaluate responses of plants. A living cell, however, is not limited to those mentioned above.

A "step of detecting a change in surface plasmon resonance angle resulting from a change in mitochondrial polarized state" in the present invention is carried out by detecting a change in surface plasmon resonance angle during a period in which the change in surface plasmon resonance angle substantially results solely from a change in mitochondrial polarized state. In a case in which one or plural agents (examples of the agents include fenofibrate, quercetin, trans-resveratrol, and Herceptin, but the agent is not limited to them) are administered to a living cell, the period in which the change in surface plasmon resonance angle substantially results solely from a change in mitochondrial polarized state is normally a period that comes after a lapse of 20 minutes or longer after the agent is administered, preferably after a lapse of 30 minutes or longer, more preferably after a lapse of 35 minutes or longer.

In a case in which an agent whose effect relating to changes in mitochondrial polarized state is considered to take a while to appear, such as siRNA inducing apoptosis, is administered to a living cell, the period in which the change in surface plasmon resonance angle substantially results solely from a change in mitochondrial polarized state is a period that comes after a lapse of 20 minutes after the effect of the agent that relates to the changes in mitochondrial polarized state appears after the agent is administered to the living cell, preferably after a lapse of 30 minutes, more preferably after a lapse of 35 minutes (e.g., if the agent is Bcl-2 siRNA inducing apoptosis, approximately one hour after the agent is administered to the living cell). The time when "the effect of the agent that relates to the changes in mitochondrial polarized state appears" varies according to the type of the agent. The time when "the effect of the agent that relates to the changes in mitochondrial polarized state appears" can be determined by confirmation using a conventional technique (e.g., fluorescence method, living-cell number measuring method), with reference to, for example, the Examples described below. Responses, other than the mitochondrial polarized state, that are detected with a surface plasmon resonance device are bindings of agents to cell membranes and the resulting changes in polarized state of the cell membrane, and the like. The responses normally subside in approximately 30 minutes after a change in an environment outside the cell, such as administration of a drug or exchange of an extracellular solution (Analytical Biochemistry 2002; 302: 28-37). Thus, if the changes in resonance angle after this period of time are observed, no response other than the changes in mitochondrial polarized state is considered to be substantially detected. Further, responses that occur in the cell after a lapse of approximately 30 minutes or longer after a change in an environment outside the cell, such as administration of an agent or exchange of an extracellular solution, are known, as a result of the study of the inventors of the present invention, to have no substantial effect on the changes in surface plasmon resonance angle (refer to the Examples) if the speed of pH change is within a preferred range (e.g., the cases of Examples 1-4 described below).

The "method of evaluating an effect of one or plural agents on a mitochondrial polarized state in a living cell" in the present invention is broadly divided into two cases. One of the cases is a method in which when an agent whose function is unknown is administered to a cell, and a change in surface plasmon resonance angle is measured to determine on the basis of its response whether the agent induces a change in mitochondrial polarized state is induced. The other one of the cases is a method in which an agent that is already determined by a fluorescence method or the like as to whether it induces a change in mitochondrial polarized state in a cell is evaluated in terms of a level of change that the agent induces in a different cell. In the former case, there is a possibility that, if a conventional technique is employed, a response other than the changes in mitochondrial polarized state is observed. On the other hand, with the method disclosed in the present Specification, no response other than the changes in mitochondrial polarized state is substantially detected.

The method of the present invention is also employable to quantify an effect of a target agent (one or plural agents) on the changes in mitochondrial polarized state by use of a slope of a graph plotted on the basis of signals from the surface plasmon resonance sensor that are recorded over time (the amount of change in surface plasmon resonance angle per time unit, that is to say, a rate of change). In this case, a period in which the changes in surface plasmon resonance angle substantially result solely from a change in mitochondrial polarized state and the detected changes in surface plasmon resonance angle are constant is specified. That is to say, a period in which a slope of a graph plotted on the basis of signals from the surface plasmon resonance sensor that are recorded over time is a substantially-straight line is specified to use the slope of the graph within the period thus specified. The length of the period thus specified is at least 1 minute, preferably 3 minutes or longer, more preferably 5 minutes or longer, even more preferably 10 minutes or longer. For more precise quantification, a rate of change in surface plasmon resonance angle during a time period in which the rate of change is plus or minus 10% or below of the rate of change during a period that includes the time period and is longer than the time period by 10% or above can be used.

Concretely, in a case in which the time period is a 5-minute period from 35 to 40 minutes, this time period is selectable if the rate of change in a period that is longer than the 5-minute period by 10% or above, i.e., a period of 5 minutes and 30 seconds or longer (e.g., a 10-minute period between 30 and 45 minutes), is plus or minus 10% or below of the rate of change in the 5-minute period from 35 to 40 minutes. In a case in which the time period is a 10-minute period from 35 to 45 minutes, this time period is selectable if the rate of change in a period that is longer than the 10-minute period by 10% or above, i.e., a period of 11 minutes or longer (e.g., a 15-minute period between 30 and 50 minutes), is plus or minus 10% or below of the rate of change in the 10-minute period from 35 to 45 minutes. As the foregoing describes, a feature of the present invention is that changes in mitochondrial polarized state in a living cell can be detected in a shorter period of time (within one hour) than that in the conventional methods. If, however, it is considered to take a while to detect the changes in mitochondrial polarized state, for instance if the speed of pH change is slow, a person skilled in the art can determine the period of time for the detection by referring to the disclose of the present Specification.

With the present invention, the rate of change in surface plasmon resonance angle may be obtained in advance with respect to an agent whose level of effect on a mitochondrial polarized state is already known, and the rate of change of the target agent may be compared with the rate of change thus obtained. Further, the level of the effect of the target agent on the mitochondrial polarized state can be analyzed quantitatively by comparing it with a calibration curve that is obtained likewise.

Examples of the agents that may cause changes in mitochondrial polarized state in living cells include (a) lipid metabolism promoting agents (fat burning agent), (b) cell-apoptosis inducing agents, (c) siRNA, (d) anticancer agents, (e) carcinogen, (f) agents that have effect on cell division activity, (g) endocrine-disrupting substances, and (h) agents that are toxic to cells. Regarding actions of such agents, it is possible to evaluate not only the actions of each agent when it is used alone but also the actions of each agent when it is used in combination. Note that the agents are not limited to compounds. For instance, the agents may be siRNA, which is involved in induction of apoptosis. To evaluate changes in mitochondrial polarized state in living cells, which changes are caused by siRNA, a time lag between administration of siRNA and emergence of an effect of si RNA that relates to changes in mitochondrial polarized state can be taken into consideration by referring to, for instance, the methods described in Examples below. The time lag is considered to be affected by the cell type and the type of the responses of the target cell.

The present invention uses a surface plasmon resonance device.

The principles of surface plasmon resonance are described as follows. In the following description, a case of using a prism is discussed. Surface plasmon resonance is a phenomenon that occurs when metal-surface plasmon is resonantly excited by evanescent waves obtained from light such as laser light. The evanescent waves can be produced by causing a total reflection in the prism so that the evanescent waves are produced on an opposite side to a side of this reflection. If there is a thin metal layer on the prism, the evanescent waves pass through the metal layer to resonantly excite surface plasmon on the other side of the metal. Factors that determine the conditions for causing the resonance include dielectric constant of a substance that forms an interface with the metal layer. Since interaction between molecules is accompanied by changes in dielectric constant, it determines the conditions for resonance. At this time, the conditions of the evanescent waves that can induce the resonance also change. Therefore, in reverse, changes in dielectric constant, and consequently bindings between molecules, can be determined by detecting changes in conditions of the evanescent waves that induce the resonance. In reality, the conditions of the evanescent waves vary according to an incident angle $\theta$ of incident laser light. The incident angle and the reflection angle are equal and are each defined as an angle to the normal line of the plate. When a resonance phenomenon occurs, the evanescent waves are affected by the substance on the metal layer to cause a rapid attenuation in intensity of the reflection light. Changes in a microscopic region of the metal layer can be determined by detecting changes in intensity of the reflection light with the use of a detector and plotting a graph of incident angles (resonance angle) at which the intensity of the reflection light is attenuated. To measure the surface plasmon resonance, a method that utilizes light diffraction with the use of a diffraction grating can be employed other than the method that utilizes total reflection of light with the use of a prism. According to the method in which the resonance angles are obtained, the dielectric constant, corresponding to es, of the measurement target defines the resonance angle $\theta$ as shown in the equation below. Changes in mitochondrial polarized state in living cells, which changes are the measurement target, cause changes in dielectric constant es. Therefore, when a stimuli causes a change in mitochondrial polarized state, the change in mitochondrial polarized state is detected by the SPR sensor in the form of a change in resonance angle $\theta$ in response to a change in dielectric constant es.

[Equation 1]

$$\sin\theta = \frac{1}{n_p} \sqrt{\frac{\varepsilon_m \cdot \varepsilon_s}{\varepsilon_m + \varepsilon_s}}$$

Further, time changes in surface plasmon resonance can be measured in the form of time changes in surface plasmon resonance angle, or time changes in intensity of the reflection light at a surface plasmon resonance angle of the initial state. Other than the foregoing detection method in which the resonance angles are obtained, it is also possible to employ a method in which, using white light as a light source for casing surface plasmon resonance excitation, changes in intensity of reflection light with either a wavelength dispersing detected light and causing a resonance phenomenon, or a constant wavelength.

An existing surface plasmon resonance device can be employed in the method of the present invention. For instance the device disclosed in JP 2005-17081 A can be employed.

The fact that the changes in mitochondrial polarized state are detected by the method of the present invention can be confirmed by various methods. For instance, the fact can be confirmed by determining correlation between the rate of change obtained in the present invention and values relating to mitochondrial polarization that are obtained by a fluorescence method, which is a conventional technique (a method that includes introducing fluorescent dye responsive to electric potential, such as cationic carbocyanine dye, into cells, and detecting, with a fluorescent microscope, a flow cytometry, a spectrophotometer, or the like, changes in fluorescence wavelength as a result of aggregation of the dye in a mitochondrion having high electric potential, and changes in fluorescence intensity in response to changes in electric potential). For more concrete methods, refer to the Examples of the present invention.

Further, as described in, for instance, Examples 3 and 4 below, a correlation is found in cells on which an apoptosis-inducing agent has acted, between the rate of change in mitochondrial polarized state obtained in the present invention and cell viability calculated by a living-cell number measuring method, which is a conventional technique. Accordingly, the method of the present invention enables easier evaluation of the action of the apoptosis-inducing agents, compared with the conventional living-cell number measuring method. This is described as follows with reference to the Examples below. For instance, in order to evaluate the action of the apoptosis-inducing agents, the conventional living-cell number measuring method needs the steps of co-culturing the apoptosis-inducing agents with the cells for 48 hours or longer, staining the cells to discriminate living cells from dead cells, and then measuring with a hemocytometer or the like. On the other hand, the method of the present invention enables the results to be obtained without labeling (without staining the cells), normally within one hour.

The method of the present invention uses living cells. An extracellular solution simply needs to ensure that the cells survive during the measurement. Examples of such solution include cellular mediums, buffer solutions, physiological salines, and sucrose solutions. The extracellular solution, however, is not limited to those mentioned above. It is preferable that the extracellular solution be a cellular medium.

With the method of the present invention, the speed of change in mitochondrial polarized state (mitochondrial membrane potential) in living cells can be controlled by controlling the pH of the extracellular solution at the time of the detection. Concretely, the speed of change in mitochondrial polarized state in living cells can be accelerated either by increasing or by reducing the pH of the extracellular solution. At this time, the speed of pH change becomes faster at faster speeds of change in mitochondrial polarized state. The speed of pH change is a rate of pH change per unit time, and it can be calculated on the basis of the period of time of the measurement and pH levels of the extracellular solution before and after the measurement. Concretely, the speed of pH change can be calculated in the way as described in Example 5 below.

If a condition in which a pH is maintained substantially constant, such as a speed of pH change (pH 0.004/min in Example 5) under a $CO_2$ concentration of 5% and a speed of pH change in a case of using an extracellular solution having a composition that is not affected by a concentration of $CO_2$ in the atmosphere, commonly used for measurement of changes in mitochondrial polarized state, is defined as a state in which a change in mitochondrial polarized state is not accelerated, it is implied that a change in mitochondrial polarized state would be accelerated under a condition of where a rate of change in pH is fast(er).(Note: It is not clear what the subject of comparison is here.) Although a relationship between the speed of pH change and the speed of change in mitochondrial membrane potential more or less varies according to cell type and a type of stimuli, a faster speed of pH change is considered suitable for detection of changes in a mitochondrial polarized state, because a speed of change in mitochondrial membrane potential becomes faster at faster speeds of pH change. A preferred pH range is considered to vary slightly according to the cell type and so on.

In a state in which the change in mitochondrial polarized state is not accelerated, it takes two hours or longer to reach a period in which the change in surface plasmon resonance angle substantially results solely from a change in mitochondrial polarized state. This length of time can be shortened by controlling the pH of the extracellular solution as described above. To control the pH of the extracellular solution, the speed of pH change of the extracellular solution is adjusted to, for instance, pH 0.005/min or above, preferably pH 0.01/min or above, and more preferably pH 0.02/min or above. The speed of change needs to fall within a range in which the living cells can survive. To control the pH of the extracellular solution, a technique that is publicly known among people skilled in the art can be employed, with consideration given to the effect on the living cells, such as gradually replacing the extracellular solution with a solution having a different pH, adding a solution having a different pH to the extracellular solution, or controlling the $CO_2$ concentration of the extracellular solution. For more concrete methods, refer to the Examples of the present invention.

Preferred Embodiment for Carrying out the Invention

The method of the present invention for detecting changes in mitochondrial polarized state in living cells can be used to evaluate properties of living cells that relate to mitochondrial polarization, such as division activity, senescence, and malignancy (whether it is a cancer cell or a normal cell). In this case, the level of a target activity or a target action can be determined by making a comparison of values, obtained by use of surface plasmon resonance, between cells that are under the same conditions (e.g. cell lines, culture solution systems, optical conditions).

The method of the present invention can be used to evaluate action of one or plural agents on living cells, which action is related to mitochondrial polarization, such as fat-burning action, apoptosis-inducing action, toxicity, and endocrine-disrupting action.

Evaluation of Cell Division Activity

To select a good cell for artificial insemination or cloning plants and animals, it is important to select a cell having high division activity. Since a mitochondrial polarized state closely relates to cell division activity, it is possible with the present invention to non-invasively select a good cell for the foregoing use by monitoring the mitochondrial polarized state without labeling (refer to Cancer Research 1998; 58 (13): 2869-2875).

Evaluation of Cell Senescence

Senescent cells have low division activity, and mitochondrial polarized states are related to such activity. Thus, cell senescence can be monitored by monitoring mitochondrial polarized states. This is also applicable to screening of drugs that activate cells to retard senescence (refer to Biological Signals and Receptors 2001; 10: 176-188).

Screening Fat-Burning Agents (anti-obesity drugs)

It is known that accumulation of excess fat is not only a symptom of obesity but also causes disease such as diabetes, cerebral apoplexy, and arteriosclerosis. In view of this, fat burning agents are highly useful as anti-obesity drugs. Further, fat burning is carried out in brown fat cells and hepatic cells. Since it is known that mitochondrial polarization or depolarization occurs in hepatic cells when fat is burnt, screening of fat-burning agents can be carried out by monitoring mitochondrial polarized states in brown fat cells (refer to Biophysical journal 2002; 82(1): 1673 part 2, and FEBS Letters 1984; 170(1): 181-185).

Diagnosis of Cancer

It is known that a mitochondrial polarized state of a cancer cell differs significantly from that of a normal cell. This is applicable to discrimination between normal cells and cancer cells (refer to Cancer Research 2005; 65(21): 9861-9867).

Screening Anticancer Agents

It is known that a mitochondrial polarized state changes, such as mitochondrial depolarization, during a method in which an anticancer agent that exhibits anticancer action by inducing apoptosis acts on cancer cells. Further, it is found that the anticancer action can be evaluated on the basis of the level of the depolarization. Thus, the screening of a candidate anticancer agent can be carried out by monitoring mitochondrial polarized states at the time when the candidate anticancer agent is administered to cancer cells (refer to Apoptosis 2005; 10: 687-705). Further, for instance, a synergistic effect produced by combined administration of the candidate anticancer agent can be determined by monitoring mitochondrial polarized states.

Evaluation of RNAi

RNAi (RNA interference) is a phenomenon that double-stranded RNA induced in a cell decomposes mRNA having a base sequence complementary to that of the double-stranded RNA. Artificial induction of double-stranded RNA using the phenomenon enables repression of a gene expression. Effect of RNAi that is considered to affect especially mitochondrial depolarization can be determined by monitoring mitochondrial polarized states. Examples of such RNAi include those involved in cell apoptosis, cell division activity, senescent, obesity, obesity-related diabetes, cerebral apoplexy, arteriosclerosis, brown fat cell, or hepatic cell. RNAi, however, is not limited to those mentioned above (for RNAi, refer to Nature 2001; 411: 494-498).

Environment Monitoring

It is known that an endocrine-disrupting substance known as an environmental hormone, such as bisphenol A, induces proliferation or apoptosis of a specific cell according to its type and/or concentration. Since cell division and apoptosis in cell proliferation can be detected by monitoring mitochondrial polarized states, the present invention can be used to detect endocrine-disrupting substances in an environment and to evaluate an effect of the substances (refer to Archives of Toxicology 2000; 74(2): 99-105, and Journal of Biological Chemistry 2005; 280(7): 6181-6196).

Evaluation of Toxicity

It is commonly known that administration of an agent that is toxic to a living cell causes rapid mitochondrial depolarization. There are cases in which toxicity of various agents is evaluated by monitoring mitochondrial polarized states with the use of a fluorescent reagent. By the present invention, toxicity can be easily evaluated without labeling (refer to Hepatology 2000; 31; 1141-1152, and Toxicological Science 2005; 86(2): 436-443). The method of the present invention for detecting changes in mitochondrial polarized state in living cells can be used to evaluate division activity, senescence, and malignancy (whether it is a cancer cell or a normal cell) of candidate living cells and to carry out the screening of the cells.

Further, the method of the present invention can be used to carry out the screening of a candidate agent (e.g., pharmaceutical candidate compounds for treatment of disease or state that relates to mitochondrial polarization). A concrete method of such screening includes the steps of:

administering a candidate agent to a living cell;
detecting, after the agent is administered to the living cell, a change in surface plasmon resonance angle resulting from a change in mitochondrial polarized state;
specifying a period in which the change in surface plasmon resonance angle substantially results solely from a change in mitochondrial polarized state (e.g., after a lapse of 20 minutes or longer after the agent is administered) and the detected change in surface plasmon resonance angle is constant (i.e., a graph of signals that are sent from the surface plasmon resonance sensor and recorded over time has a slope of that is a substantially-straight line), and obtaining a rate of change in surface plasmon resonance angle during the period thus specified; and
selecting a candidate agent on the basis of the rate of change in surface plasmon resonance angle thus obtained.

Concrete examples of the disease and conditions that relate to mitochondrial polarization include: those related to cell division activity; those related to senescence, obesity or obesity-related diabetes, cerebral apoplexy or arteriosclerosis, or mitochondrial polarization in brown fat cells or hepatic cells; and those related to cancer and apoptosis.

[EXAMPLES]

The following describes Examples of mitochondrial polarized state monitoring.

Device

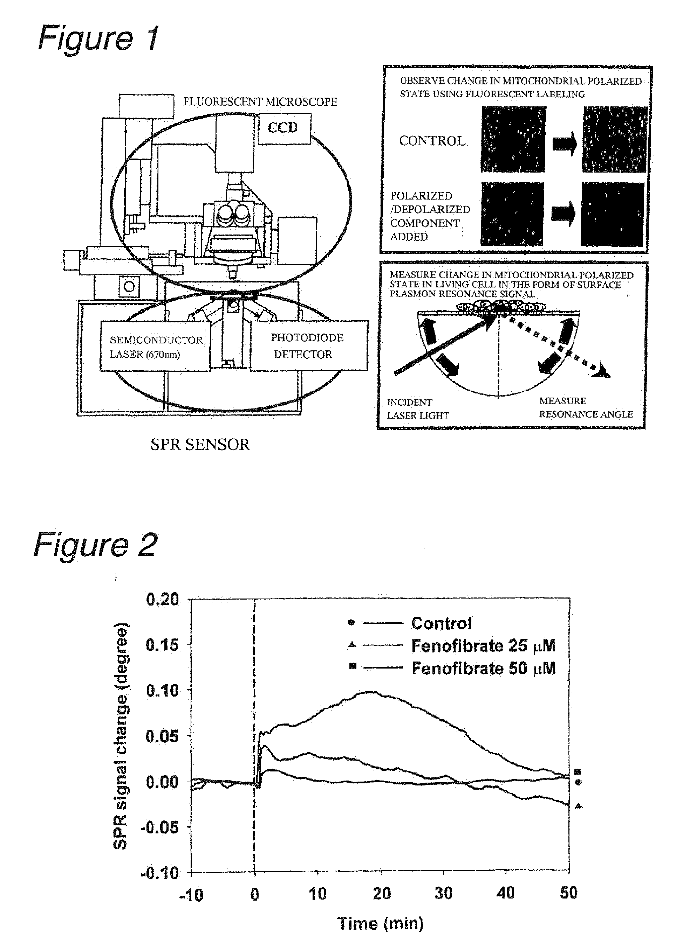

Measurement of SPR signals and fluorescent microscopic observation were carried out using a device having a fluorescent microscope located above living cells, which cells are a target of the measurement, and an SPR sensor located under the living cells. Figure 1 shows a diagram of the device. An optical system of Kretschmann configuration was used as an SPR sensor. BK7 (with a refractive index of 1.51) was used as a prism. A semiconductor laser (with a wavelength of 670 nm, an output of 3 mW, and a beam diameter of 1 mm) was used as a light source. A silicon photodiode detector was used as a detector. The measurement was carried out in the atmosphere (with 20% $O_2$ and 0.035% $CO_2$), unless otherwise stated.

The fluorescent microscopic observation was carried out with the use of Axioplan 2 (Carl Zeiss) as a microscope, a 75-W xenon lamp as a light source, and NTE/CCD Detector MicroMAX-512BFT (Princeton Instruments) as a detector. Images were obtained and analyzed with the use of fluorescence analysis software, MetaFluor Imaging System Ver. 4.65 (Universal Imaging).

EXAMPLE 1

[Evaluation of Lipid Metabolism Promoting Agent]

Human liver cancer cells HepG-2 were used as test cells. A liquid medium EMEM containing 100 μM nones-

sential amino acid, 50 units/mL penicillin, 50 µg/mL streptomycin, and 10% (v/v) FBS was prepared, at 37°C with a $CO_2$ concentration of 5%, to be used as a complete medium, and the cells were pre-cultured in the complete medium and then used in the testing. Fenofibrate (2-[4-(4-Chlorobenzoyl)phenoxy-2-methylpropanoic acid isopropyl ester) was used as a lipid metabolism promoting agent. Lipid metabolism activity was evaluated at the time when 25 µM fenofibrate was added and at the time when 50 µM fenofibrate was added. DMSO (dimethyl sulfoxide) (DMSO is contained at a final concentration of 0.1% (v/v) after it is added to the medium) was used as a solvent. Further, only DMSO was added to a control in such a way that the final concentration was adjusted to 0.1% (v/v).

1. Evaluation of lipid metabolism activity using SPR

Following pre-culture, the test cells were removed from the petri dish, and a concentration of the test cells in the complete medium was adjusted to 2 x 10$^6$ cell/mL. On a plate, a 100 µL suspension of the test cells was dropped and cultured for 18 hours at 37°C with the $CO_2$ concentration of 5%. After 18 hours, the plate was placed on a prism of an SPR sensor, and on the plate was filled 5 mL EMEM. Then, measurement was started. Ten minutes after the measurement was started, replacement with a medium containing fenofibrate was carried out, and the measurement was continued for another 50 minutes. Consequently, as shown in Figure 2, changes (-) were observed in the SPR signals resulting from mitochondrial polarization due to activation of lipid metabolism, especially about 20 minutes after the reagent was added.

2. Monitoring a mitochondrial polarized state using fluorescent reagent JC-1

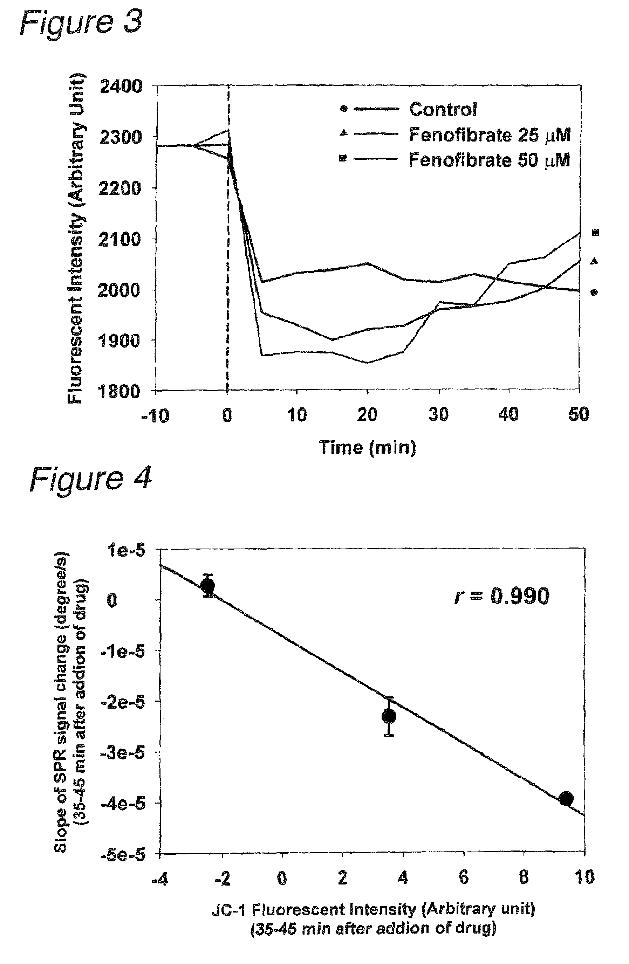

Following pre-culture, the test cells were removed from the petri dish, and the concentration of the test cells in the complete medium was adjusted to 2 x 10$^6$ cell/mL. On the plate, a 100 µL suspension of the test cells was dropped and cultured for 18 hours at 37°C with the $CO_2$ concentration of 5%. After 18 hours, replacement with 100 µL EMEM (DMSO was contained at a final concentration of 0.1%) containing 2.5 µM JC-1 iodide was carried out, and the plate was left in a $CO_2$ incubator (at 37°C with the $CO_2$ concentration of 5%) for 20 minutes. After 20 minutes, the medium on the plate was replaced with EMEM to remove the medium containing JC-1 iodide. Then, it was placed at the measuring section of the device and filled with 5 mL EMEM, and the measurement was carried out at 37°C. The fluorescence intensity was observed at 630-fold magnification. An excitation filter was used to transmit 485 nm plus or minus 20 nm. To detect fluorescence, a set of filters that transmitted 515-565 nm and a set of filters that transmitted 575-640 nm were used. Ten minutes after the measurement was started, EMEM was removed, and replacement with a medium containing fenofibrate was carried out, and then the measurement was continued. A medium containing 0.1% (v/v) DMSO was used as a control. Consequently, as shown in Figure 3, an increase in fluorescence intensity resulting from mitochondrial polarization due to activation of lipid metabolism was observed, especially about 20 minutes after the reagent was added. Further, as shown in Figure 4, a high correlation (r = 0.990) between the time rate of change in SPR signal and the level of mitochondrial polarization was observed, showing that changes in SPR signal resulted from changes in mitochondrial polarized state.

EXAMPLE 2

[Evaluation of an apoptosis-inducing agent]

Human pancreas cancer cells MIA PaCa-2 were used as test cells, and cultured in a liquid medium EMEM containing 100 µM nonessential amino acid, 50 units/mL penicillin, 50 µg/mL streptomycin, and 10% (v/v) FBS, at 37°C with the $CO_2$ concentration of 5%.
Quercetin and *trans*-resveratrol were used as apoptosis-inducing agents. Rutin, a non-apoptosis-inducing agent, was used as a negative control. Solutions of a thousandfold concentration (10 mM, 25 mM, 50 mM, and 100 mM) were prepared for respective final concentrations, at the time of use, of 10 µM, 25 µM, 50 µM, and 100 µM, by use of dimethyl sulfoxide (DMSO) as a solvent, and the solutions thus prepared were stocked.
Further, Herceptin, a commercially available anticancer agent, was used as an apoptosis-inducing agent. Solutions of a thousandfold concentration were prepared for respective final concentrations, at the time of the use, of 1 µg/mL, 10 µg/mL, and 100 µg/mL, by use of physiological saline as a diluent solvent, and the solutions thus prepared were stocked.

1. Measurement of SPR signals

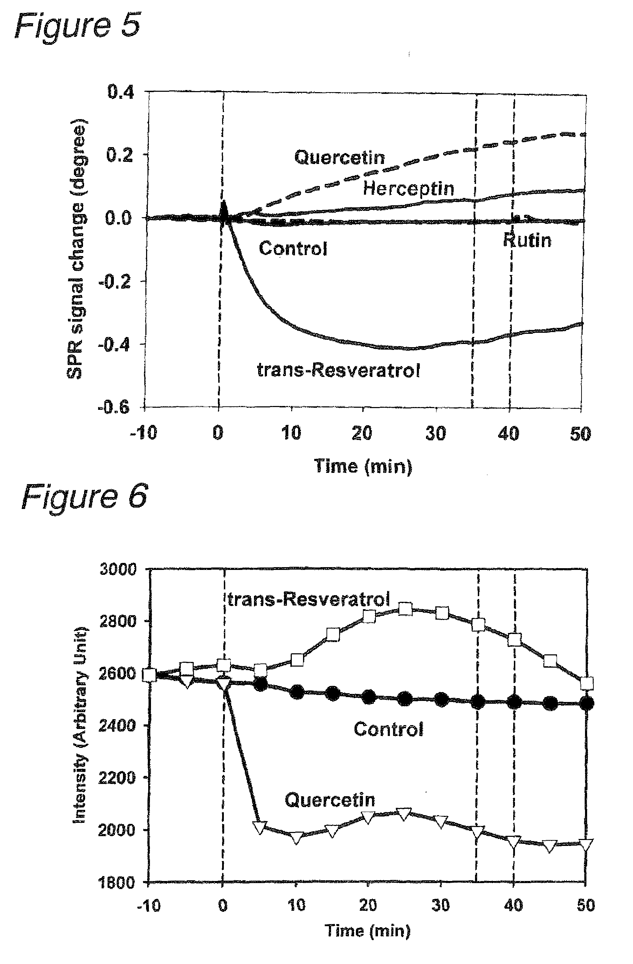

SPR signals were measured as follows. A plate to which the cells adhered was placed on the prism of the SPR sensor via matching fluid (with a refractive index of 1.51), and on the plate was filled 5 mL EMEM. Then, measurement of SPR signals was started. Ten minutes after the measurement was started, EMEM was removed, and either a replacement with new 5 mL EMEM (DMSO used as a solvent of a phenol component was contained at a final concentration of 0.1% (v/v)) containing 100 $\mu$M of any one of quercetin, *trans*-resveratrol, rutin, and Herceptin, or a replacement with 5 mL EMEM containing only 0.1% DMSO as a control was carried out, and then the measurement was continued for another 50 minutes. Consequently, as shown in Figure 5, changes (+) in SPR signal resulting from mitochondrial depolarization caused by the apoptosis-inducing agent were observed, especially 35 minutes after the drug was administered.

2. Monitoring mitochondrial polarized states using fluorescent reagent JC-1

After the cells were removed from the petri dish, a suspension of the cells was prepared with the use of a liquid medium, and a concentration of the cells was adjusted to 2 x $10^6$ cells/mL with the liquid medium. After the adjustment, 100 $\mu$L of the cell suspension was dropped onto the plate and cultured for 20 hours at 37°C with the $CO_2$ concentration of 5%.

After 20 hours, the plate was rinsed with 5 mL PBS to remove cells that did not adhere to the medium. As a fluorescence indicator for measuring the mitochondrial polarized state, 100 $\mu$L EMEM (containing DMSO having a final concentration of 0.1%) containing 5 $\mu$M JC-1 iodide was dropped, and it was left in the $CO_2$ incubator (at 37°C with the $CO_2$ concentration of 5%) for 30 minutes. After 30 minutes, the plate was rinsed with 5 mL PBS, placed at the measuring section of the device, and filled with 5 mL EMEM. Then, the measurement was carried out at 37°C. The fluorescence intensity was observed at 200-fold magnification. An excitation filter was used to transmit 485 nm plus or minus 20 nm. To detect fluorescence, a set of filters that transmitted 515-565 nm and a set of filters that transmitted 575-640 nm were used. Ten minutes after the measurement was started, EMEM was removed, and either a replacement with new 5 mL EMEM (DMSO used as the solvent was contained at the final concentration of 0.1% (v/v)) containing 100 $\mu$M quercetin or *trans*-resveratrol, or a replacement with 5 mL EMEM containing only 0.1% DMSO as a control was carried out, and then the measurement was continued for another 50 minutes. Consequently, as shown in Figure 6, the results of time-dependent mitochondrial depolarization caused by the respective apoptosis-inducing agents were observed.

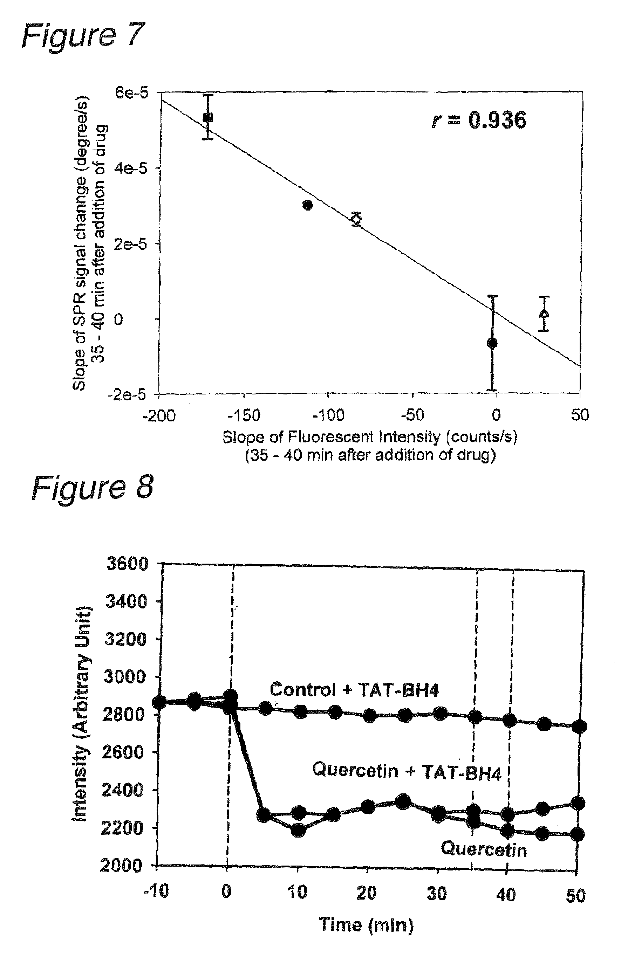

To confirm that the changes in signals obtained by the SPR sensor as shown in Examples 1-3 resulted from mitochondrial depolarization, the following comparison was made using Example 3 as an exemplary case. The amount of change in SPR signal during a period of approximately 5 minutes (in the case of Example 3, a period between 35 minutes and 40 minutes after the reagent was administered) following convergence of cellular responses that were not suitable for monitoring the mitochondrial polarized state, such as binding of the reagent to a surface of the cell membrane immediately after the reagent and changes in polarized state of the cell membrane, was compared with the amount of change in mitochondrial polarized state that was obtained during the same period by fluorescent labeling. As a result, a high correlation (r = 0.936) was observed as shown in Figure 7.

3. Testing apoptosis inhibition by suppressing mitochondrial depolarization

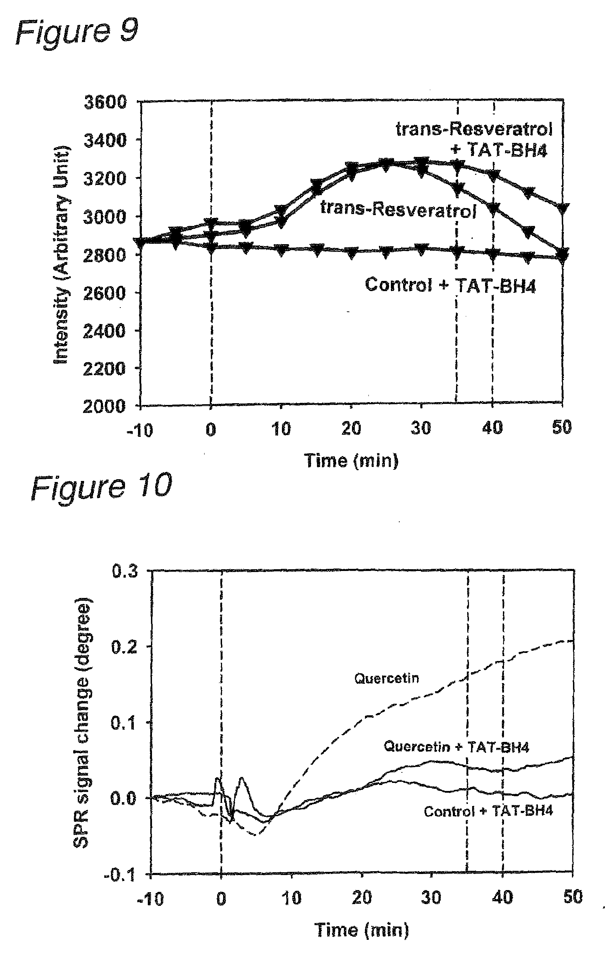

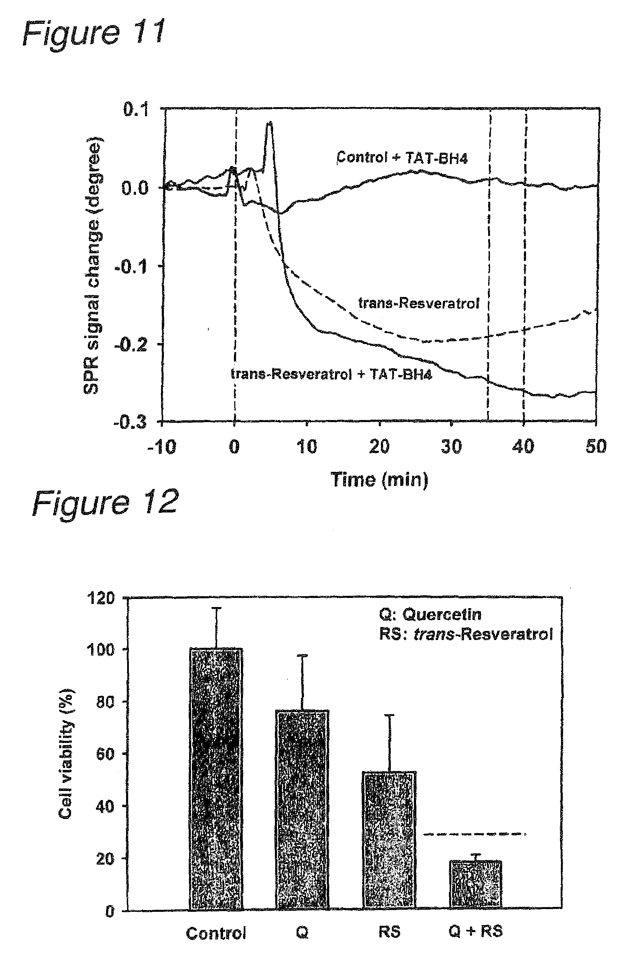

The fact that the detection of the changes in SPR signal meant detection of changes in mitochondrial polarized state was determined by use of a reagent inhibiting mitochondrial depolarization at the time when apoptosis was induced into the cancer cells by quercetin and *trans*-resveratrol. The mitochondrial test cells MIA PaCa-2 were cultured on the plate under the same conditions, at 37°C with the $CO_2$ concentration of 5%. Bcl-xL BH4 (4-23) (Human), Cell-Permeable (hereinafter, "TAT-BH4"), which bound to a mitochondrial membrane and inhibited mitochondrial membrane depolarization and cytochrome c release, was used as an apoptosis inhibitor. Fifteen minutes before the measurement was started, the liquid medium on the plate was removed and replaced with 100 $\mu$L EMEM containing 100 nM inhibitor, and it was incubated for 15 minutes at 37°C with the $CO_2$ concentration of 5% to introduce the inhibitor into the cells. Alternatively, a replacement with 100 $\mu$L EMEM, as a control, in which PBS (-), used as the solvent of the inhibitor, was contained by the same amount was carried out. The plate to which the cells adhered was placed at the measuring section of the device, and on the plate was filled 5 mL EMEM. Measurement of the fluorescence intensity and SPR signals was started in the same manner as described above. Ten minutes after the measurement was started, EMEM was removed, and either a replacement with new 5 mL EMEM (DMSO was contained as a solvent of a phenol component at the final concentration of 0.1% (v/v)) containing 100 $\mu$M quercetin or *trans*-resveratrol, or a replacement with 5 mL EMEM containing only 0.1% DMSO as a control was carried out, and then the measurement was continued for another 50 minutes. Consequently, in the same manner as the inhibition of mitochondrial depolarization confirmed by fluorescence labeling as shown in Figures 8 and 9, it was confirmed that suppression of signal change also occurred in the changes in SPR signal, so that it was confirmed that the signals detected by the SPR sensor were the changes in mitochondrial polarized state, as shown in Figures 10 and 11.

EXAMPLE 3

[Evaluation of effect produced by combined use of plural agents]

A synergistic effect of combined use of apoptosis-inducing agents (agent having antitumor activity) was evaluated. Quercetin and *trans*-resveratrol were used as agents known to have a synergistic effect. The synergistic effect of the combined use of them on apoptosis is discussed in Mouria, M. et al. Food-derived polyphenols inhibit pancreatic cancer growth through mitochondrial cytochrome c release and apoptosis. Int. J. Cancer. 98, 761-769 (2002).

1. Evaluation of combined use of plural agents on the basis of a survival rate calculated by determining the number of cells (standard method)

The effect of combined use of plural agents was evaluated by a method including co-culturing a test agent with the cells, and counting the number of reduced cells by apoptosis. Human pancreas cancer cells MIA PaCa-2 were used as the test cells. A cell suspension of $5 \times 10^4$ cells/mL was prepared with the use of EMEM (Eagles' minimum essential medium) containing 10% (v/v) FBS, 50 units/mL penicillin, 50 $\mu$g/mL streptomycin, and 100 $\mu$M nonessential amino acid, and 5 mL of the cell suspension was poured into each 6-cm petri dish and pre-cultured at 37°C with the $CO_2$ concentration of 5%. After 24 hours, either a replacement with new 5 mL EMEM (DMSO used as the solvent of the phenol component was contained at the final concentration of 0.1% (v/v)) containing 25 $\mu$M quercetin, 25 $\mu$M *trans*-resveratrol, or both 25 $\mu$M quercetin and 25 $\mu$M *trans*-resveratrol in addition to the medium, or a replacement with 5 mL EMEM containing only 0.1% (v/v) DMSO as a control was carried out, and then culturing was carried out. Forty eight hours after the medium was replaced, trypan blue staining was carried out, and then the number of cells was counted with a hemocytometer. Comparison was carried out for the respective conditions, with 100 representing the number of cells in the control.

2. Measurement of SPR signals

SPR angles were measured with the above-described SPR sensor (tilting sensor) at the temperature of 37.0°C. Likewise in the measurement of the survival rate, a cell suspension of $2 \times 10^6$ cell/mL was prepared, and 100 $\mu$L of the cell suspension was dropped onto a gold plate for SPR measurement and cultured for 20 hours at 37°C with the $CO_2$ concentration of 5%. After 20 hours, the plate was placed on the prism of the SPR sensor, and on the plate was filled 5 mL EMEM. Then, measurement was started. Ten minutes after the measurement was started, either a replacement with new 5 mL EMEM (DMSO used as the solvent of the phenol component was contained at a final concentration of 0.1% (v/v)) containing 25 $\mu$M quercetin, 25 $\mu$M *trans*-resveratrol, or both 25 $\mu$M quercetin and 25 $\mu$M *trans*-resveratrol, or a replacement with 5 mL EMEM containing only 0.1% (v/v) DMSO as a control was carried out, and then the measurement of the changes in SPR angle was continued for another 50 minutes.

Results

Figure 12 shows the survival rate calculated on the basis of the number of cells counted, with 100 representing the control. The survival rate under the condition in which quercetin was administered alone was 76.1%, and the survival rate under the condition in which *trans*-resveratrol was administered alone was 56.2%. On the other hand, the survival rate of the cancer cells under the condition in which quercetin and *trans*-resveratrol were administered in combination was 17.8%, showing that a greater antitumor activity effect than a sum (corresponding to the survival rate of 32.3%) of the antitumor activity effect of quercetin administered alone and that of *trans*-resveratrol administered alone was obtained. The synergistic effect of combined use of quercetin and *trans*-resveratrol on antitumor activity was confirmed from the results.

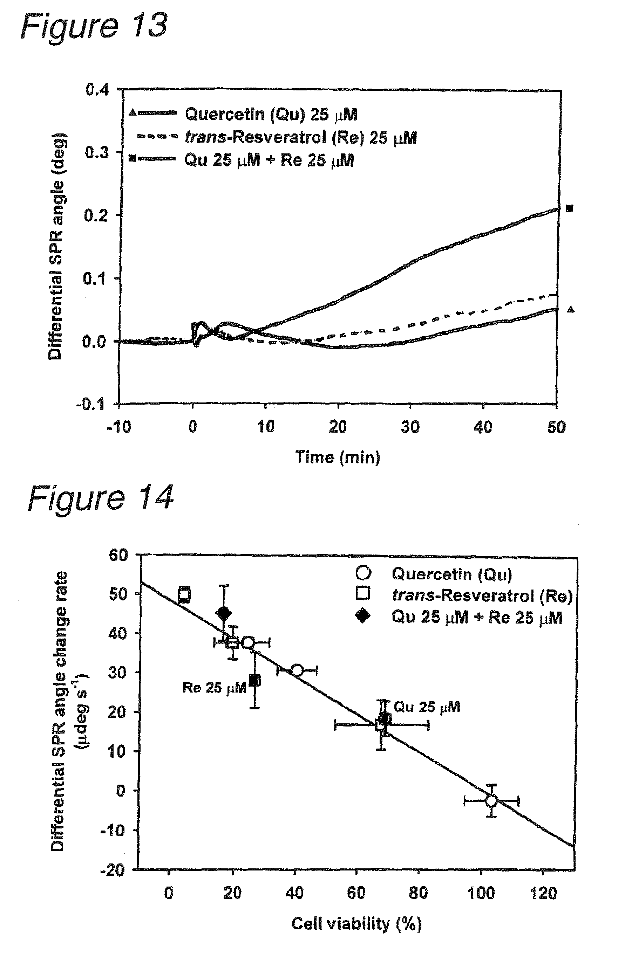

Figure 13 shows the results of measurement of changes in SPR angle over time in the case in which quercetin was administered alone, in the case in which *trans*-resveratrol was administered alone, and in the case in which quercetin and *trans*-resveratrol were administered in combination. In the cases in which either quercetin or *trans*-resveratrol was administered alone, the change in SPR angle was approximately 0.05 degrees 50 minutes after the administration. In the case in which quercetin and *trans*-resveratrol were administered in combination, a change in resonance angle by an angle of 0.2 degrees or greater was observed. As described in Example 2, the changes in SPR angle obtained as a result of the administration of quercetin and *trans*-resveratrol were changes (depolarization) in mitochondrial membrane potential at the time when apoptosis was induced into the cancer cells. The present Example shows that changes in mitochondrial membrane potential that correspond to the synergistic effect on induction of apoptosis into cancer cells can be detected also in the case in which two kinds of agents are administered in combination.

Further, to determine a correlation between the changes in SPR angle shown in Figure 13 and the results obtained by a standard method (survival rate calculated by determining the number of cells), the speed (deg/sec) of change in SPR angle was calculated on the basis of the changes in SPR angle during the period between 35 minutes and 40 minutes after the reagent was administered. This value shows a correlation with the survival rate obtained by the standard method (Figure 14). Measuring the changes in mitochondrial membrane potential in living cells by use of the SPR method also makes it possible to promptly and quantitatively predict the synergistic effect of combined use of two kinds of reagents on antitumor activity.

EXAMPLE 4

[Evaluation of RNA interference (RNAi)]

RNA interference (RNAi) was evaluated by use of small interfering RNA (siRNA). Bcl-2, a molecule considered to act in a cell to suppress apoptosis, was knocked down by use of RNAi in order to induce apoptosis. Changes in mitochondrial polarized state at that time were monitored using SPR, and effectiveness of siRNA was evaluated on the basis of the amount of change. Induction of apoptosis using siRNA of Bcl-2 is discussed in Feng, L.F. et al. Bcl-2 induced apoptosis and increased sensitivity to 5-fluorouracil and HCPT in HepG2 cells. J. Drug Targeting 14, 21-26 (2006).

1. Evaluation of effectiveness of siRNA on the basis of a survival rate calculated by determining the number of cells (standard method)

Effectiveness of siRNA was evaluated by counting the number of cells reduced by apoptosis as a result of the knocking down of Bcl-2. Human pancreas cancer cells MIA PaCa-2 were used as the test cells. A cell suspension of 1 x $10^4$ cells/mL was prepared with the use of EMEM containing 10% (v/v) FBS, 50 units/mL penicillin, 50 $\mu$g/mL strep-tomycin, and 100 $\mu$M nonessential amino acid, and 1 mL of the cell suspension was poured into each 24-well petri dish and pre-cultured at 37°C with the $CO_2$ concentration of 5%. After 24 hours, in addition to the medium mentioned above, a replacement of 50 nM Bcl-2 siRNA (for sequences, refer to Genes Dev. 17(7) 832-837 (2003)), 100 nM Bcl-2 siRNA, or, as a control, 1 mL EMEM containing a transfection reagent, used to introduce siRNA, at the same concentration as that at the time of inducing siRNA was carried out, and then culturing was carried out (SignalSilence™ Bcl-2 siRNA Kit (Human Specific) of Cell Signaling Technology was used in accordance with protocol of the kit). Forty eight hours after the medium was replaced, trypan blue staining was carried out, and the number of cells was counted using a hemocytometer. Comparison was carried out for the respective conditions, with 100 representing the number of cells in the control.

2. Measurement of SPR signals

SPR angles were measured at the temperature of 37.0°C with the use of a tilting SPR sensor as the device. Likewise in the measurement of the survival rate, 100 $\mu$L cell suspension of 2x$10^6$ cells/ml was dropped onto a gold plate for SPR measurement, and cultured for 20 hours at 37°C with the $CO_2$ concentration of 5%. After 20 hours, a replacement with new 5 mL EMEM containing 50 nM Bcl-2 siRNA, 100 nM Bcl-2 siRNA, or, as a control, a transfection reagent to be used to induce siRNA was carried out. To calm disturbance due to coexistence of a transfection buffer, and to take into consideration a time lag between the administration of the reagent and the time when the mitochondrial membrane potential became high enough to reach a detection sensitivity and frequent enough to be detected, it was left still for another one hour at 37°C with the $CO_2$ concentration of 5%, and then response of the test cells was measured in the form of changes in SPR angle for 50 minutes.

Results

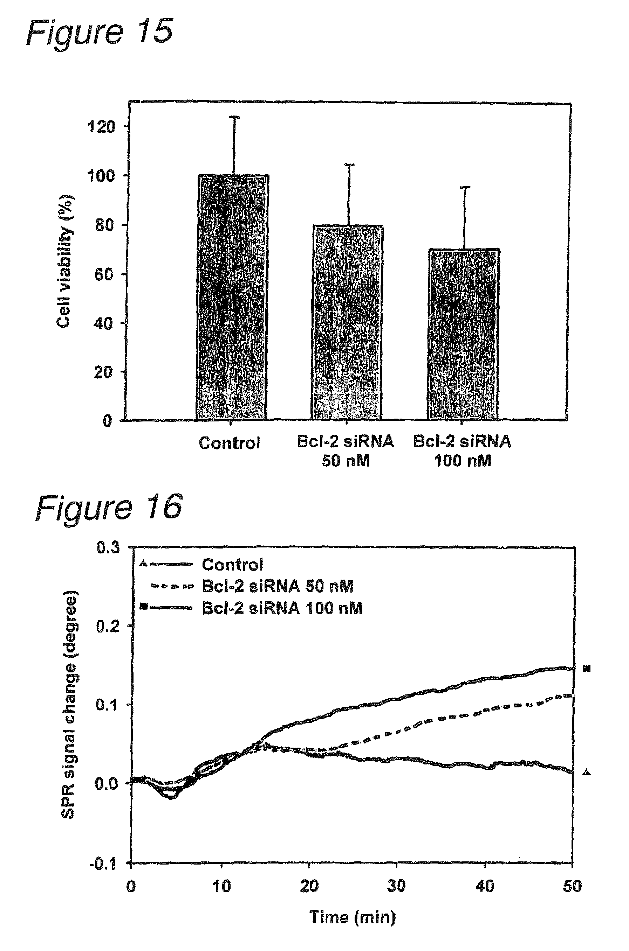

Figure 15 shows the results of the standard method (the survival rate calculated by counting the number of cells, with 100 representing the control). Contrary to the conditions of the control, the survival rate under the conditions that 50 nM Bcl-2 siRNA was administered was approximately 80%, and the survival rate under the conditions that 100 nM Bcl-2 siRNA was administered was approximately 70%. It was confirmed from those results that the induction of apoptosis by siRNA caused the number of cells to decrease.
Figure 16 shows the results of changes in SPR angle over time one hour after the administration of siRNA of the respective concentrations and after the administration of the transfection reagent alone. In all of the conditions, stable changes in SPR angle were observed 15 minutes after the measurement was started. Under the condition of 50 nM Bcl-2 siRNA and the condition of 100 nM Bcl-2 siRNA, increase in SPR angle was observed which indicated changes in mitochondrial membrane potential (depolarization). This shows that apoptosis induced as a result of knocking down Bcl-2, which is an apoptotic suppressor, by use of RNAi can be detected in a form of changes in mitochondrial membrane potential.

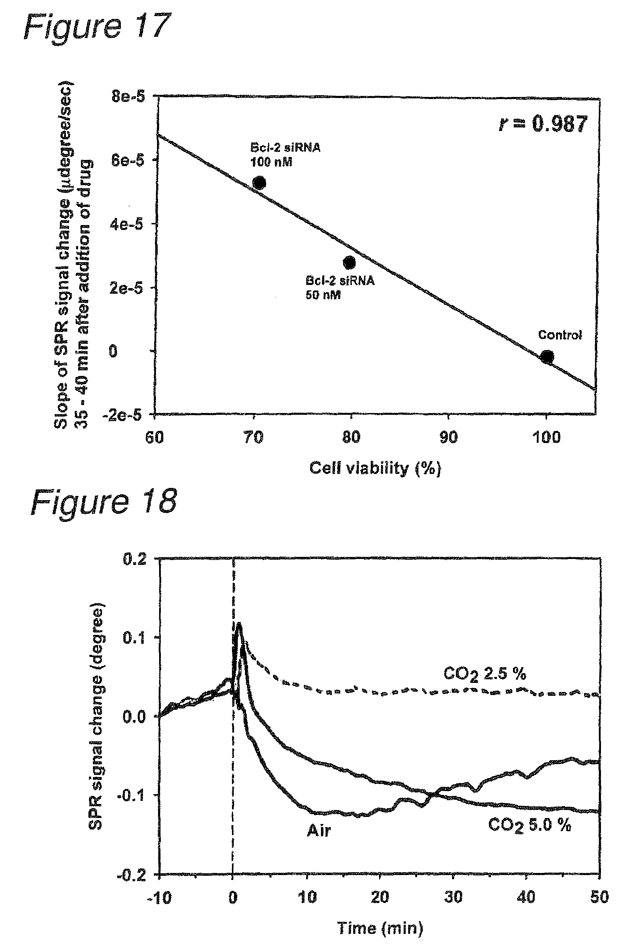

Further, to obtain a correlation between the changes in SPR angle shown in Figure 16 and the results obtained by a standard method (survival rate calculated by determining the number of cells), the speed (deg/sec) of change in SPR angle was calculated on the basis of the changes in SPR angle during the period between 35 minutes and 40 minutes after the measurement was started. This value shows the correlation with the survival rate obtained by the standard method (Figure 17). The foregoing results show that use of the SPR method enables significantly prompt quantitative evaluation of RNAi.

EXAMPLE 5

[Method of accelerating the changes in mitochondrial membrane potential]

The following describes a method of controlling the speed of change in mitochondrial membrane potential in living cells by controlling the pH of an extracellular solution used in the measurement. The pH was changed by controlling the concentration of $CO_2$ in the atmosphere outside the extracellular solution (buffer solution) to change a balance in the extracellular solution.

The method was carried out at the temperature of 37.0°C by use of the above-described SPR sensor (tilting sensor equipped with a fluorescent microscope) as the device. Human pancreas cancer cells MIA PaCa-2 were used as the test cells. The cells were cultured in a liquid medium EMEM containing 100 $\mu$M nonessential amino acid, 50 units/mL penicillin, 50 $\mu$g/mL streptomycin, and 10% (v/v) FBS, at 37°C with the $CO_2$ concentration of 5%. To induce changes in mitochondrial membrane potential, frans-resveratral, which is an apoptosis-inducing reagent, was used. During the measurement, a pH change, $CO_2$ concentration, and $O_2$ concentration were controlled by adjusting a flow rate of each gas by use of a flowmeter.

1. Evaluation of SPR signals

Measurement of SPR signals was carried out as follows. A plate to which cells adhered was placed on the prism of an SPR sensor via matching fluid (refractive index 1.51), and on the plate was filled with 5 mL EMEM. Then, the measurement of SPR signals was started. Ten minutes after the measurement was started, EMEM was removed, and a replacement with new 5 mL EMEM containing 100 $\mu$M *trans*-resveratrol was carried out, and then the measurement was carried out for 50 minutes. The measurement was carried out in the air (in the atmosphere with 20% $O_2$ and 0.035% $CO_2$), in 2.5% $CO_2$, and in 5.0% $CO_2$. The $CO_2$ concentration was adjusted by mixing, with a flowmeter, the air (the atmosphere) and $CO_2$ gas supplied from a $CO_2$ tank. The results are shown in Figure 18.

2. Monitoring a mitochondrial polarized state using fluorescent reagent JC-1

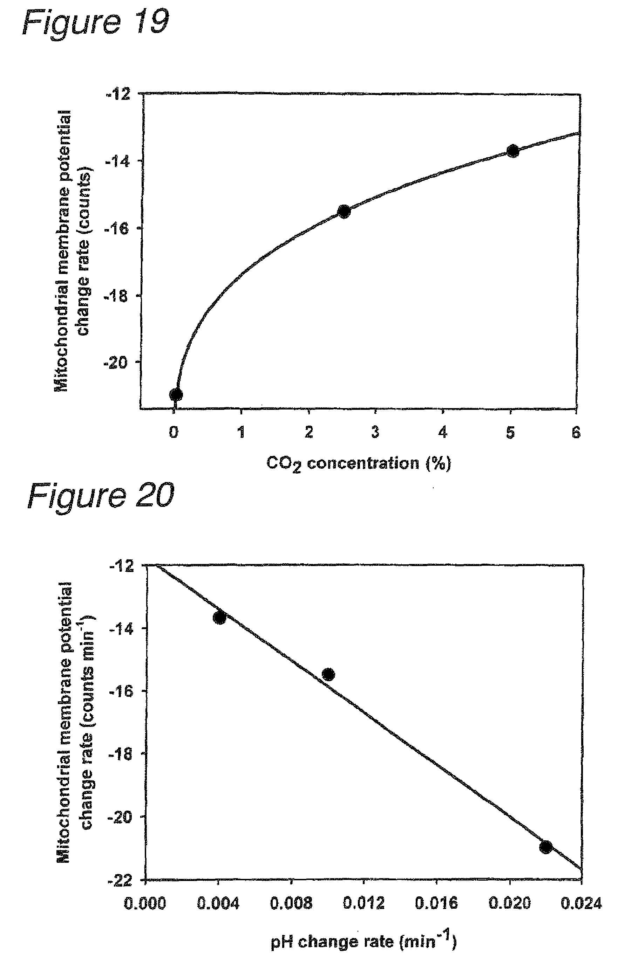

After the cells were removed from the petri dish, a suspension of the cells was prepared with the use of a liquid medium, and a concentration of the cells was adjusted to 2 x $10^6$ cells/mL. After the adjustment, 100 $\mu$L of the cell suspension was dropped onto a plate and cultured for 20 hours at 37°C with the $CO_2$ concentration of 5%. After 20 hours, the plate was rinsed with 5 mL PBS to remove cells that did not adhere to the medium. As a fluorescence indicator for measuring the mitochondrial polarized state, EMEM 100 $\mu$L (DMSO was contained at the final concentration of 0.1%) containing 5 $\mu$M JC-1 iodide was dropped, and it was left in the $CO_2$ incubator (37°C, $CO_2$ concentration of 5%) for 30 minutes. After 30 minutes, the plate was rinsed with 5 mL PBS, placed at the measuring section of the device, and filled with 5 mL EMEM. Then, likewise in the section "1. Evaluation of SPR signals" above, the measurement was carried out at 37°C in the air (in the atmosphere with 20% $O_2$ and 0.035% $CO_2$), in 2.5% $CO_2$, and in 5.0% $CO_2$. The fluorescence intensity was observed at 200-fold magnification. An excitation filter was used to transmit 485 nm plus or minus 20 nm. To detect fluorescence, a set of filters that transmitted 575-640 nm was used. Ten minutes after the measurement was started, EMEM was removed, and new 5 mL EMEM (DMSO used as the solvent was contained at the final concentration of 0.1% (v/v)) containing 100 $\mu$M *trans*-resveratrol was carried out, and the measurement was continued for another 50 minutes. Changes in mitochondrial polarized state were observed that were obtained by fluorescent labeling during a period of approximately 5 minutes (in the case of Example 5, a period between 35 minutes and 40 minutes after the reagent was administered) following convergence of cellular responses that were not suitable for monitoring the mitochondrial polarized state, such as binding of the reagent to a surface of the cell membrane immediately after the reagent and changes in polarized state of the cell membrane. An integrated value of fluorescence intensity corresponding to the level of the mitochondrial membrane potential was calculated on the basis of images of observed fluorescence during the respective periods of time. Figure 19 shows a relationship between obtained changes in integrated value over time (the speed of change in mitochondrial membrane potential during the period from 35 to 40 minutes) and the $CO_2$ concentration.

Results

As shown in Figure 18, a decrease in mitochondrial membrane potential that was detected in the form of increase in changes in SPR angle was accelerated as the $CO_2$ concentration increased, in the following order: the air (in the atmosphere with 20% $O_2$ and 0.035% $CO_2$) > $CO_2$ 2.5% > $CO_2$ 5.0%. The same results were also obtained in the measurement of changes in mitochondrial membrane potential by use of the fluorescent reagent, as shown in Figure 19. The pH of the medium (buffer solution) used as the extracellular solution was 7.3 before the measurement was started. On the other hand, the pH of the medium was approximately 8.4 in the atmosphere, approximately 7.8 at 2.5% $CO_2$, and approximately 7.5 at 5% $CO_2$, after the measurement was carried out for 50 minutes. The pH changes are linear. The speed of medium pH change obtained from the results is approximately pH 0.022/min in the atmosphere, approximately pH 0.01/min at 2.5% $CO_2$, and approximately pH 0.004/min at 5% $CO_2$. Figure 20 shows a relationship between the speed of pH change and the speed of change in mitochondrial membrane potential during the period between 35 and 40 minutes. Figure 20 shows that the speed of pH change correlates with the speed of change in mitochondrial membrane potential.

If the speed of pH change (pH 0.004/min in Example 5) at the $CO_2$ concentration of 5%, which is an experimental system commonly used in the measurement of mitochondrial membrane potential, is defined as a state in which the change in mitochondrial membrane potential is not accelerated, it is implied that the change in mitochondrial membrane potential would be accelerated under a condition of a faster speed of pH change. The relationship between the speed of pH change and the speed of change in mitochondrial membrane potential more or less varies according to the type of the cells or stimuli. A faster speed of pH change is considered suitable for detection of changes in mitochondrial polarized state, because the speed of change in mitochondrial membrane potential becomes faster at faster speeds of pH change.

**Claims**

1. A method of detecting a change in mitochondrial polarized state in a living cell comprising the step of:

   detecting, by use of a surface plasmon resonance device, a change in surface plasmon resonance angle resulting from the change in mitochondrial polarized state of the living cell.

2. A method of detecting a change in mitochondrial polarized state in a living cell comprising the steps of:

   administering one or plural agents to the living cell; and
   detecting, after the one or plural agents are administered to the living cell, a change in surface plasmon resonance angle resulting from the change in mitochondrial polarized state.

3. The method of Claim 2, wherein, in the step of detecting the change in surface plasmon resonance angle resulting from the change in mitochondrial polarized state, the change in surface plasmon resonance angle is detected only during a period in which the change in surface plasmon resonance angle results solely from the change in mitochondrial polarized state.

4. The method of Claim 2, wherein, in the step of detecting the change in surface plasmon resonance angle resulting from the change in mitochondrial polarized state, the change is detected only during a period that comes after a lapse of 20 minutes or longer after the one or plural agents are administered, preferably after a lapse of 30 minutes or longer, more preferably after a lapse of 35 minutes or longer.

5. The method of Claim 3 or 4, further comprising the step of specifying a period in which the detected change in surface plasmon resonance angle is constant, which period has the length of at least 1 minute, preferably 3 minutes or longer, more preferably 5 minutes or longer, even more preferably 10 minutes or longer, and obtaining a rate of change in surface plasmon resonance angle during the period thus specified.

6. The method of Claim 5, wherein the rate of change in surface plasmon resonance angle during the period specified is plus or minus 10% or below of the rate of change in surface plasmon resonance angle during a period that includes the period specified and is at least 10% longer than the period specified.

7. The method of Claim 4, comprising the step of comparing an obtained rate of change with a rate of change in surface plasmon resonance angle of an agent whose level of effect on the mitochondrial polarized state is known.

8. The method of Claim 1 or 2, wherein the living cell is a cultured cancer cell.

9. The method of Claim 1 or 2, wherein the change in mitochondrial polarized state in the living cell is detected after at least one of, preferably after any one of, the following steps (a) to (h), preferably (a)-(d):

   (a) administering a lipid metabolism promoting agent to the living cell;
   (b) administering a cell-apoptosis inducing agent to the living cell;
   (c) administering siRNA to the living cell;
   (d) administering an anticancer agent to the living cell;
   (e) administering a carcinogen to the living cell;
   (f) administering an agent to the living cell, the agent having an effect on cell division activity;
   (g) administering an endocrine-disrupting substance to the living cell; and
   (h) administering an agent to the living cell, the agent being toxic to the cell

10. The method of Claim 1 or 2, wherein a speed of pH change of an extracellular solution during the step of detecting the change in surface plasmon resonance angle is pH 0.005/min or above, preferably pH 0.01/min or above, more preferably pH 0.02/min or above.

## Figure 1

SPR SENSOR

## Figure 2

## Figure 3

## Figure 4

*Figure 5*

*Figure 6*

## Figure 7

## Figure 8

*Figure 9*

*Figure 10*

*Figure 11*

*Figure 12*

## Figure 13

## Figure 14

## Figure 15

## Figure 16

## Figure 17

## Figure 18

## Figure 19

## Figure 20

<table>
<tr><td colspan="2" align="center">**INTERNATIONAL SEARCH REPORT**</td><td>International application No.<br>PCT/JP2006/324960</td></tr>
</table>

A. CLASSIFICATION OF SUBJECT MATTER
*C12Q1/02*(2006.01)i, *G01N21/27*(2006.01)i, *G01N21/64*(2006.01)i, *G01N33/15*
(2006.01)i, *G01N33/50*(2006.01)i, *G01N33/543*(2006.01)i

According to International Patent Classification (IPC) or to both national classification and IPC

B. FIELDS SEARCHED

Minimum documentation searched (classification system followed by classification symbols)
C12Q1/02, G01N21/27, G01N21/64, G01N33/15, G01N33/50, G01N33/543

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho   1971-2007   Toroku Jitsuyo Shinan Koho   1994-2007

Electronic data base consulted during the international search (name of data base and, where practicable, search terms used)
BIOSIS(STN) CAplus(STN) MEDLINE(STN) JMEDPlus(JDream2)

C. DOCUMENTS CONSIDERED TO BE RELEVANT

| Category* | Citation of document, with indication, where appropriate, of the relevant passages | Relevant to claim No. |
|---|---|---|
| Y | JP 2005-017081 A (Japan Science and Technology Agency), 20 January, 2005 (20.01.05), & WO 2005/001472 A1 | 1-10 |
| Y | MOURIA, M. et al.Food-derived polyphenols inhibit pancreatic cancer growththrough mitochondrial cytochrome C release and apoptosis.Int. J. Cancer., 2002, Vol. 98, No. 5, p. 761-769. | 1-10 |
| Y | WO 2004/042079 A1 (UNISEARCH LTD), 21 May, 2004 (21.05.04), & EP 1563099 A1    & US 2006/166208 A1 & JP 2006-518986 A | 1-10 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>12 March, 2007 (12.03.07) | Date of mailing of the international search report<br>20 March, 2007 (20.03.07) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/324960

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-085089 A (Japan Science and Technology Corp.), 26 March, 2002 (26.03.02), & US 2005/0100904 A1 & EP 001469309 A1 & WO 2003/060514 A1 | 1-10 |
| A | HAIL, N. JR., Mitochondria: A novel target for the chemoprevention of cancer.Apoptosis, 2005 Augusut, Vol. 10, No. 4, p. 687-705 | 1-10 |
| A | HIDE, M. et al., Real-time analysis of ligand-induced cell surface andintracellular reactions of living mast cellsusing a surfaceplasmon resonance-based biosensor. Analytical Biochemistry, 2002, Vol. 302, p. 28-37 | 1-10 |
| P,X | KOSAIHIRA, A. et al., Identification of molecular mechanism of apoptosis viamitochondrial pathway detected by Surface Plasmon Resonance. 20th IUBMB International Congress of Biochemistry and MolecularBiology and 11th FAOBMB Congress, 2006, p. A14667 (3P-A-352) | 1-10 |

Form PCT/ISA/210 (continuation of second sheet) (April 2005)

REFERENCES CITED IN THE DESCRIPTION

**Patent documents cited in the description**

- JP 2002085089 A

- JP 2005017081 A

**Non-patent literature cited in the description**

- *Biological Signals and Receptors,* 2001, vol. 10, 176-188
- *Anticancer: Apoptosis,* 2005, vol. 10, 687-705
- *Toxic agent: Hepatology,* 2000, vol. 31, 1141-1152
- *Toxicological Sciences,* 2005, vol. 86 (2), 436-443
- *Lipid metabolism: Biophysical journal,* 2002, vol. 82 (1), 1673
- *FEBS Letters,* 1984, vol. 170 (1), 181-185
- *Analytical Biochemistry,* 2002, vol. 302, 28-37
- *Cancer Research,* 1998, vol. 58 (13), 2869-2875
- *Biophysical journal,* 2002, vol. 82 (1), 1673
- *Cancer Research,* 2005, vol. 65 (21), 9861-9867
- *Apoptosis,* 2005, vol. 10, 687-705

- *Nature,* 2001, vol. 411, 494-498
- *Archives of Toxicology,* 2000, vol. 74 (2), 99-105
- *Journal of Biological Chemistry,* 2005, vol. 280 (7), 6181-6196
- *Hepatology,* 2000, vol. 31, 1141-1152
- *Toxicological Science,* 2005, vol. 86 (2), 436-443
- **MOURIA, M. et al.** Food-derived polyphenols inhibit pancreatic cancer growth through mitochondrial cytochrome c release and apoptosis. *Int. J. Cancer.,* 2002, vol. 98, 761-769
- **FENG, L.F. et al.** Bcl-2 induced apoptosis and increased sensitivity to 5-fluorouracil and HCPT in HepG2 cells. *J. Drug Targeting,* 2006, vol. 14, 21-26
- *Genes Dev.,* 2003, vol. 17 (7), 832-837